(19) Europäisches Patentamt
European Patent Office
Office européen des brevets

(11) **EP 2 493 394 B1**

(12) **EUROPEAN PATENT SPECIFICATION**

(45) Date of publication and mention
of the grant of the patent:
**16.10.2013 Bulletin 2013/42**

(21) Application number: **10793043.0**

(22) Date of filing: **29.10.2010**

(51) Int Cl.:
***A61B 17/11*** (2006.01)

(86) International application number:
**PCT/IB2010/054902**

(87) International publication number:
**WO 2011/051909 (05.05.2011 Gazette 2011/18)**

(54) **END-TO-END JOINT FOR CONNECTING END ZONES OF BODY DUCTS**

ENDVERBINDUNGSSTÜCK ZUR VERBINDUNG DER ENDBEREICHE VON KÖRPERDURCHFÜHRUNGEN

JONCTION BOUT À BOUT DESTINÉE À RELIER DES ZONES D'EXTRÉMITÉ DE CONDUITS CORPORELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2009 IT BO20090713**

(43) Date of publication of application:
**05.09.2012 Bulletin 2012/36**

(60) Divisional application:
**13183736.1**

(73) Proprietor: **NEWMAN MEDICAL KFT.**
**1146 Budapest (HU)**

(72) Inventor: **BORGHI, Enzo**
**I-40054 Budrio (IT)**

(74) Representative: **Conti, Marco**
**Bugnion S.p.A.**
**Via di Corticella, 87**
**40128 Bologna (IT)**

(56) References cited:
**US-A1- 2004 249 400      US-A1- 2005 149 075**
**US-A1- 2008 269 784**

## Description

Technical Field

This invention relates to an end-to-end joint for connecting end zones of body ducts.

In other words, the invention relates to a vascular joint for anastomosis operations in which two hollow structures consisting of blood vessels or, more generally, ducts for human body fluids, are surgically connected.

Since the structures connected are two end portions of a duct for body fluids (for example, a blood vessel, a lymphatic vessel or other type of duct), the operation is called end-to-end anastomosis, precisely because the portions are connected frontally or "end-to-end" in order to restore flow to the blood vessel or duct.

Background Art

In practice, the surgeon might for example have to cut a body duct in order to perform a particular operation; at a later stage, the end portions (or free ends) of the cut duct must be connected in order to restore the continuity of the duct.

A first prior art solution for an end-to-end vascular joint (see document WO2001/72232 by the same Inventor as the present) basically comprises a first and a second joining member, both consisting of annular members configured to encompass externally the respective ends of the blood vessel or duct.

Each of these members is connected by hook-shaped elements, or staples, to the corresponding end portion of one of the blood vessels or ducts to be connected.

These two joining members are in turn joined to each other by a connector element adapted to cover the end zones of the blood vessels or ducts joined.

This joining structure, however, has a major disadvantage and that is that its metallic parts (namely, the staples) are positioned inside the blood vessel (that is to say, in contact with the fluid flowing in the duct the joint is applied to): that means there is a serious risk of creating fluid clots inside the duct which might lead to the formation of thrombi, as well as a further risk of infection.

A second prior art solution for an end-to-end vascular joint, which was intended to improve the one described above, is disclosed in patent document WO2006/043129 by the same Inventor as the present. Likewise, patent document US2005/0149075A by the same Inventor discloses an end-to-end anastomosis device according to the preamble of present claim 1.

In the second solution, each of the two joining members is composed of two units: an annular guide member (made of a plastic material) having a plurality of through holes uniformly spaced round its circumference, and an annular staple equipped with a plurality of pointed legs.

The staple is designed to be coupled to the annular guide member by inserting the legs in the guide holes in such a way that it is substantially coaxial with the guide member. The annular guide member is made in such a way that when the legs are inserted into the guide holes, the legs are bent inwards at an angle so they are oriented according to the directrices of a cone: this allows the staple legs to penetrate the wall of the blood vessel or duct from the outside inwards but without going right through the wall of the blood vessel or duct and thus without coming into contact with the blood (or other body fluid flowing in the duct).

Obviously, in this case, too, the connection between the two joining members is secured by a connector element.

This solution also has some disadvantages, however:

- the annular member with the guide holes for the staple legs has large radial dimensions relative to the dimensions of the blood vessel or duct;
- the cost of each metal staple with the pointed legs is very high since the staple is made by a mechanical process that involves removing material from a solid block (usually cylindrical);
- the presence, for each joining member, of an element with guide holes into which must be inserted the rods associated with another part constitutes a complication for the surgeon who is applying the joint;
- the joint creates a rigid vascular connection which restrains, instead of following the pulsations of the blood vessel or the peristaltic movements of other body ducts it might be applied to.

Moreover, in the case of end-to-end vascular joints, the technical solutions adopted involve turning inside out, or everting, the edges of the end portions to be connected and then applying the joint to the everted edges of the duct.

These solutions are particularly disadvantageous because eversion of the end portions of the duct to be connected creates zones of stagnation of the fluid in the duct, leading to necrosis (with the risk of infections or other complications) of the everted portions connected, which are no longer washed by blood (where the body duct concerned is a blood vessel).

Disclosure of the Invention

This invention therefore has for an aim to overcome the above mentioned disadvantages by providing an end-to-end joint for connecting the end zones of body ducts, adapted to avoid eversion of the end zones to be connected and to prevent contact between any parts of the joint and the fluid circulating in the duct and such as to minimize the dimensions (especially the radial dimensions) of the joint itself.

Another aim of the invention is to provide an end-to-end joint for connecting the end zones of body ducts and whose application by the surgeon is made particularly easy, quick and precise.

Another aim of the invention is to provide an end-to-end joint for connecting the end zones of body ducts and which is particularly easy and inexpensive to make.

In light of this, the invention also provides a method for making the joint.

Another aim of the invention is to provide an end-to-end joint for connecting the end zones of body ducts and which follows the pulsating or peristaltic movements of the duct it is applied to.

Another aim of the invention is to provide an end-to-end joint for connecting the end zones of body ducts and which makes it easier to monitor the state of health of the person it is implanted in.

These aims are fully achieved by the joint and methods for making the joint according to the invention as characterized in the appended claims

More specifically, the end-to-end joint according to the invention, for connecting the end zones of body ducts, comprises two joining members and one connector.

According to the invention, each of the joining members comprises two rings substantially equal in diameter and joined to each other by plastically deformable arms. The first ring is equipped with pointed rods which, when the rings are pushed axially towards each other in such a way as to guide the deformation of the arms, penetrate the wall of the duct to be connected and place the joining member in a stapling condition.

The rods are guided by the second ring (inside which they are operatively inserted) in such a way as to bend towards the central axis of the second ring at an angle such that they remain within the thickness of the duct wall.

The second ring also features openings which act in conjunction with tabs on the connectors to connect the two joining members to each other. The openings also make it possible to view the duct underneath. Further, the openings facilitate penetration of the rods into the duct walls, since a predetermined pressure created inside the duct during application of the joint causes the duct wall to swell outwards at the openings.

According to another aspect of the invention, both of the rings and the connector are open, that is to say, they have a break in them, to form a sort of elastic system which makes it possible to follow the pulsations or peristaltic movements of the ducts.

According to another aspect of the invention, an end-to-end vascular joint is made from a flat strip by removing material from the flat strip (preferably by chemical etching) and then bending the machined strip into the shape of a ring to form the joining member.

Brief Description of the Drawings

The technical features of the invention according to the above mentioned aims are clearly described in the claims below and its advantages are apparent from the detailed description which follows, with reference to the accompanying drawings, which illustrate a preferred non-limiting example embodiment of the invention and in which:

- Figure 1 is an exploded perspective view of an end-to-end joint according to the invention, for connecting end zones of body ducts;
- Figure 2 is a perspective view showing the joint of Figure 1 in a compact assembled state prior to its application;
- Figure 3 is a cross section, with some parts cut away in order to better illustrate others, showing one of the joining members forming part of the joint of Figure 1 in a configuration where it is stapled to the duct;
- Figure 4 is a cross section showing the joint of Figure 1 in a configuration where it connects the two duct portions;
- Figure 5 is a plan view from above showing a portion of cut strip used to make the joining rings forming part of the joint according to the invention;
- Figure 6 is a plan view from above showing a portion of cut strip used to make a connector forming part of the joint according to the invention.

Detailed Description of the Preferred Embodiments of the Invention

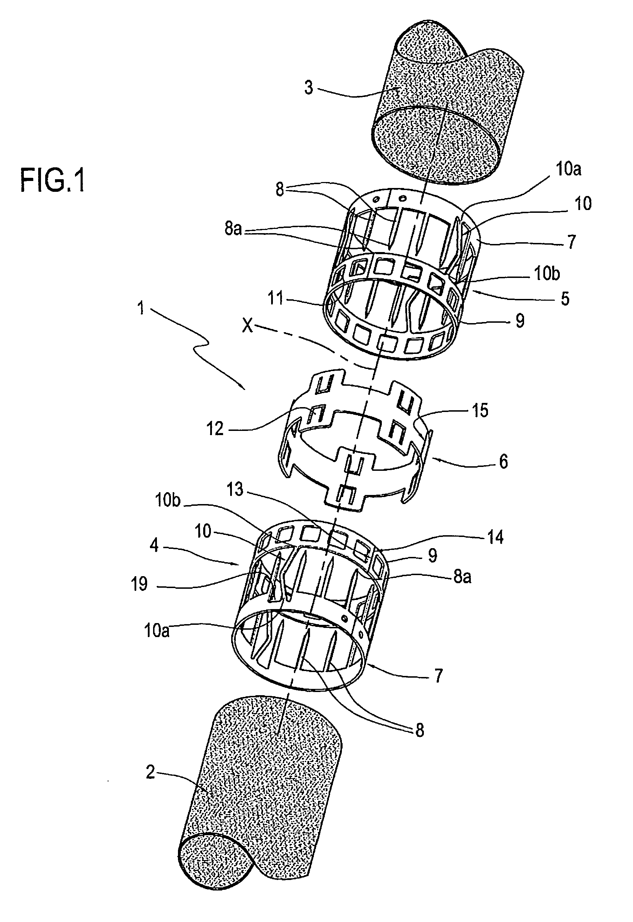

With reference to the accompanying drawings, in particular Figures 1 to 4, the end-to-end joint according to the invention, denoted in its entirety by the numeral 1, is used to connect an end zone 2 to an end zone 3 of the same body duct or, if necessary, of another duct (such as a vein, an artery or a stretch of intestine, in particular of the human body).

The joint 1 basically comprises a first and a second joining member 4, 5 that can be associated with the corresponding end zones 2, 3 of the duct to be joined, and a connector 6 that can be associated with the first and second joining members 4, 5 in order to connect them to each other when the latter are associated with the end zones 2, 3 of the duct.

As may be observed in Figures 1 to 3, each of the joining members 4 and 5 comprises: a first ring 7 and a second ring 9.

More in detail, the first ring 7 is equipped with a plurality of rods 8 positioned along directrices of the first ring 7 itself.

Preferably, the rods 8 are made as a single part with the first ring 7; also, the free ends 8a of the rods 8 are preferably tapered towards the second ring 9, that is to say, the ends are pointed so that they can penetrate a wall of the duct 2, 3 and are directed towards the second ring 9.

The second ring 9 is coaxial with the first ring 7 and has a diameter D9 which is not greater than the diameter D7 of the first ring 7, (preferably equal but possibly also slightly smaller without thereby reducing the effectiveness of the invention).

The rings 7, 9 of each joining member 4, 5 are configured to encompass externally the above mentioned duct end zones 2, 3.

According to the invention, each of the joining members 4 and 5 comprises at least two arms 10 for joining the two rings 7 and 9.

Each of the arms 10 has a first end 10a connected to the first ring 7 and a second end 10b connected to the second ring 9 to position the rings 7, 9 at a predetermined axial distance, with the rods 8 of the first ring 7 projecting towards the second ring 9.

The arms 10 are plastically deformable between an open position where the rings 7 and 9 are at said predetermined axial distance, and a bent position where the rings 7 and 9 are close together and the rods 8 are positioned inside the second ring 9 interacting with the rods 8 to bend them in such a way that they converge towards an axis X of the rings 7 and 9 in order to penetrate a wall of the duct (or rather, of the end zones 2, 3 of the duct).

It should be noted that the bent position of the arms 10 corresponds to a stapling position of the joining member 4, 5.

It should also be noted that the rings 7 and 9 are substantially cylindrical in shape, which means they define a plurality of directrices parallel to the axis X.

In the embodiment illustrated, the two rings 7 and 9 have a connecting line 13, 14 on the surface of them, along one directrix. In this embodiment, therefore, the rings 7, 9 are closed rings.

In light of this, it should be noted that the invention also contemplates an alternative embodiment (not illustrated in the drawings).

In effect, according to another aspect of the invention, the two rings 7 and 9 of each joining member 4, 5 have a break in them at the respective connecting line 13, 14 which defines two edges movable towards and away from each other to follow the pulsation of the duct 2, 3 when the joining member 4, 5 is applied to the corresponding duct 2, 3.

In light of this, it should be noted that, in the embodiment illustrated, these edges are rigidly connected (for example by laser welding).

Alternatively, the edges 13 and 14 might be connected temporarily using a bio-absorbable material which initially keeps the joining member in the position where it stably encompasses the duct 2, 3 and then, after a certain length of time, releases the edges defined by the connecting line 13 and 14 so that the rings 7 and 9 are left free to adapt to the pulsations of the fluid inside the duct.

A simpler alternative is to keep the edges free by configuring an open ring from the outset.

As regards the arms 10, there are, as illustrated, three of them and, in any case, at least three of them.

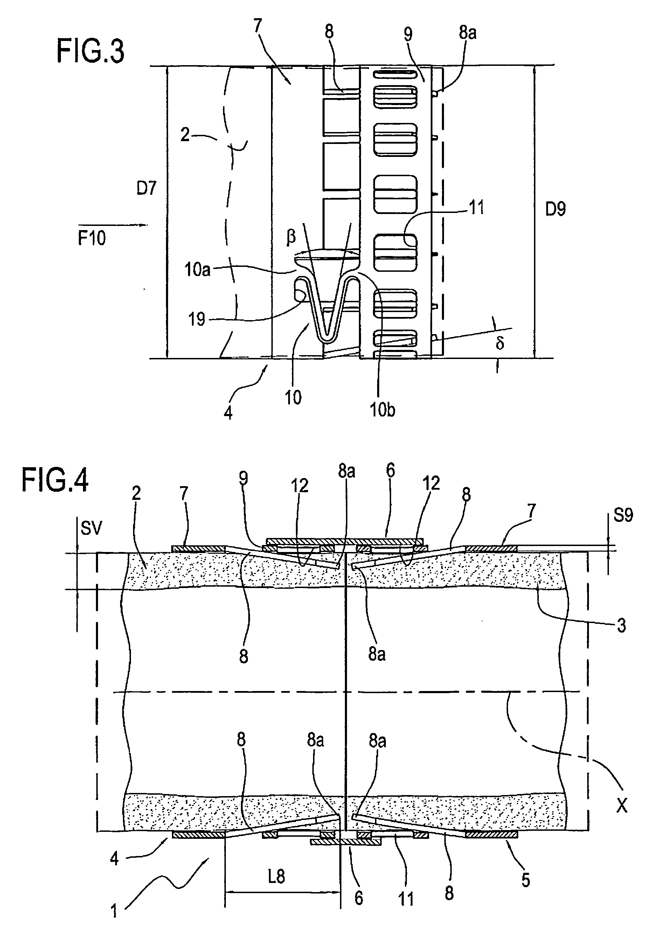

Thus, as shown in Figure 3, the arms 10 are deformable between the open position, where the rings 7 and 9 are at the predetermined axial distance, and the bent position (see Figure 3) where the rings 7 and 9 are near each other in the close-packed configuration, while the rods 8 are positioned inside the second ring 9 and are inserted in the duct wall and held within the latter without protruding to the inside of the duct where the fluid flows.

Thus, as the second ring 9 moves close to the first ring, it interacts with the rods 8 in such a way as to bend them so they converge towards the axis X and penetrate the wall of the duct 2 and 3 (see Figure 3).

In other words, therefore, the bent position of the arms 10 illustrated in Figure 3 corresponds to a stapling position of the joining members 4 and 5.

More in detail, each arm 10 comprises preferably two portions of equal length which are connected to each other uninterruptedly: that way, each arm 10 makes (that is, those portions of each arm make) an angle $\alpha$ whose amplitude varies according to the predetermined or predeterminable axial distance between the first ring 7 and the second ring 9.

Further, the two portions of each arm 10 lie in a plane tangent to the first and second rings 7 and 9 and are deformable in response to a force applied to the rings 7, 9 in axial direction (see arrow F10, Figure 3) move them close to each other into a position where the two rings 7 and 9 and the arms 10 are in a close-packed configuration.

The two portions of each arm 10 in the close-packed configuration make an angle $\beta$; this angle preferably falls within a range between 5° and 25°, and more preferably, within a range between 5° and 15°.

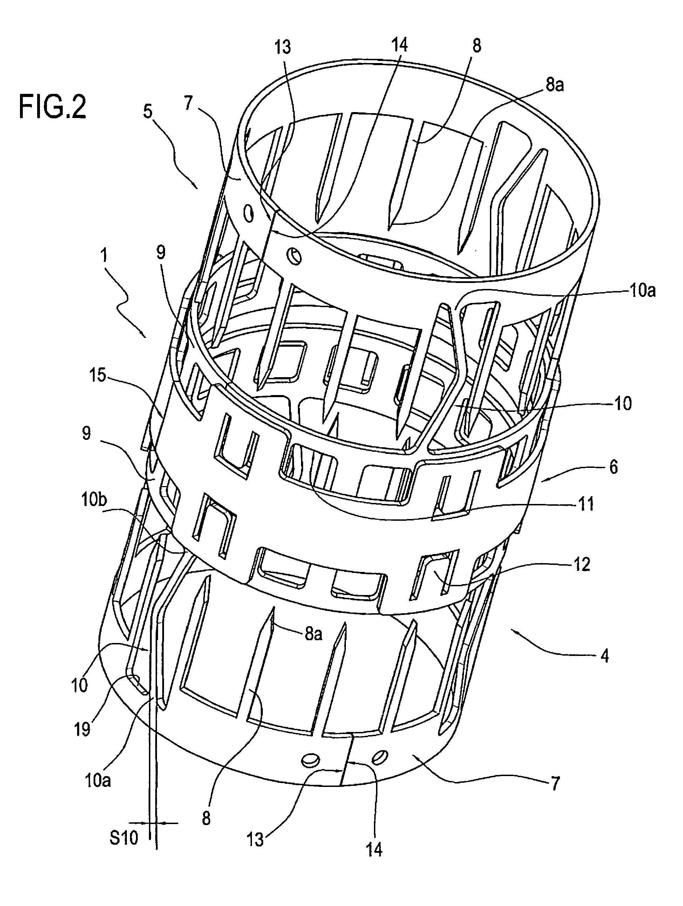

As clearly shown in Figure 2, the portion 10a of each arm 10 is connected to the first ring 7 in a portion of the first ring 7 itself, provided with a hollow 19. This creates an extension of the arm 10 portion into the hollow 19. This extension advantageously prevents the close-packed configuration of each arm 10 from reaching too small an angle (for example near 0°, that is, zero degrees) with the risk of creating on the vertex of the bent arm 10 (that is, on the central joining zone of the two arm portions) a weak zone where the arm 10 is liable to break.

Preferably, also, the first and second rings 7 and 9, the rods 8 and the arms 10 are made of the same material, for example, surgical steel.

This advantageously makes the joint particularly simple and inexpensive to produce, using the method described further on in this specification.

As stated above, when the arms 10 are in the open position, the rods 8 are within the axial dimensions defined by the two rings 7 and 9, with the pointed ends directed towards the inside edge of the second ring 9.

Preferably, the length L8 of the rods 8 is greater than the sum of the axial extension E9 of the second ring 9 plus twice the thickness S10 of the arms 10 (in a plane tangential to the outside surface of the ring, as indicated in Figure 2) .

That way, the rods 8 come into contact with the second ring 9 which causes them to bend towards the axis X and to penetrate the wall of the duct 2 or 3 by a predetermined amount (see Figure 3).

Thanks to the particular configuration described, this advantageously guarantees that the pointed rods 8 penetrate well into the wall of the duct 2 or 3.

To this we must add that, to ensure that the rod 8 penetrates into the wall of the duct 2 or 3 from the outside without going through to the inside of the duct 2 or 3, the length of each rod 8 preferably satisfies a dimensional constraint expressed by the following formula (which, in practice, also determines an angle of inclination δ of the rods when in the close-packed configuration, defined by the bending of the rods 8).

$$L8 \le SV \cdot \csc\left(arctg\ \frac{S9}{2S10}\right)$$

In the above formula (as also visible in Figure 3):

- L8 is the length of the rod 8;
- SV is the thickness of the part of the duct 2 or 3;
- S9 is the radial thickness of the second ring 9;
- S10 is the thickness of each arm 10.

Preferably, the section size of the arms 10 is greater than that of the rods 8.

In effect, the rods 8 are configured to bend easily in the close-packed (stapling) configuration.

The thicker arms 10, on the other hand, make it possible to prevent unwanted deformation of the arms 10 themselves during application of the joining member (thus avoiding the risk of the rings 7 and 9 accidentally moving apart and causing the rods 8 to be straightened).

As illustrated in the accompanying drawings, the second ring 9 also comprises a plurality of openings 11 located along its circumference and passing through from one side to another in order to make the outside wall of the duct 2 or 3 visible when the joining member 4, 5 is applied to the respective end zone of the duct 2 or 3.

The openings 11 have a twofold function of making the end zone of the respective duct 2 or 3 visible, as just stated, and also of affording an anchoring zone for the above mentioned connector 6.

In effect, the connector 6 comprises a plurality of tabs 12 configured to interact with the openings 11 of the second ring 9 in order to form a snap fit between the connector 6 and the joining members 4 and 5.

In other terms, the tabs 12 are flexible and each of them can be fitted into a respective opening 11 on the second ring 9.

The connector 6, in the shape of a ring, presents tabs 12 alternated with empty spaces to allow a free view of a respective opening 11.

In particular, the tabs are positioned on both sides so as to be able to lock the two second rings 9 of the joining members 4 and 5.

Preferably, like each of the joining members 4 and 5, the connector 6 also has a connecting line 15 along a directrix on its surface.

As with the joining members 4 and 5 whose embodiments are described above, so the connector 6 might also be embodied in the following ways:

- the edges of the connector 6 defined by the connecting line 15 and joined (for example by laser welding), in which case the connector 6 is a closed-ring connector;
- the edges of the connector 6 defined by the connecting line 15 and joined by bio-absorbable elements, in which case the connector 6 is an open-ring or delayed/programmed opening connector.

In the second embodiment, the edges of the connector 6 are movable towards and away from each other in order to follow a pulsation of the duct 2 and 3 when the connector 6 is applied to the duct.

It should be noted that the fact that the end-to-end vascular joint comprises an annular structure with a break along a directrix to allow elastic deformation of the joint to adapt to the movements (pulsations or peristalsis) of the duct it is applied to, can be used for end-to-end vascular joints of any type.

This invention thus provides a vascular joint which originally comprises an open-ring structure.

According to another aspect of the invention, the joint 1 comprises a sensor 16 for detecting the pressure of the fluid in the duct.

The sensor 16 is associated preferably with the connector 6 of the vascular joint 1.

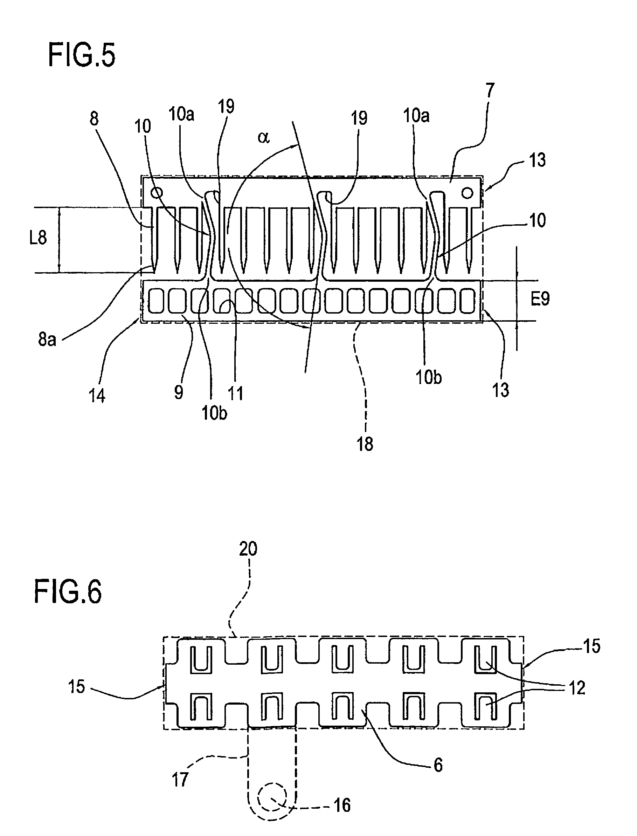

More specifically, by way of an example only, the sensor 16 for detecting the pressure may be located on a protrusion 17 on the connector 6 (drawn with a broken line in Figure 6).

It should be noted that the sensor 16 may advantageously be applied to a vascular joint of any type.

This invention thus provides a vascular joint which originally comprises a pressure sensor.

This invention also provides a method for applying the above described end-to-end joint 1 to a duct.

The method comprises the following steps:

- preparing the first and second joining members 4 and 5 and a connector 6 for joining the joining members 4 and 5;
- applying the first joining member 4 to the first end zone 2 of the duct;
- applying the second joining member 5 to the second end zone 3 of the duct;

- associating the connector 6 to the two joining members 4 and 5 in order to join them.

The step of preparing each of the joining members 4 and 5 comprises first moving the rods 8 towards the second ring 9 in such a way as to position the free ends of the rods 8 inside the second ring 9.

This advantageously makes it possible to avoid the risk that during application of the joining member 4, 5 to the duct, the rods 8 may not be positioned on the outside of the second ring 9 or may collide with it.

Application of the two joining members involves moving the two rings 7 and 9 close together by applying a force (using an applicator of substantially known type, not described since it is outside the scope of this invention) in order to plastically deform the arms 10 so that the rods 8 penetrate into the walls of the ducts 2 and 3 by interaction of the second ring 9 with the rods 8 (see Figure 3).

Lastly, application involves associating the connector 6 with the two joining members by a snap fit to engage the tabs 12 with the respective openings 11 on the second rings 9 of each joining member 4 and 5.

This invention also provides a method for making a vascular joint.

The method for making the joint 1 described above is extremely quick and practical and comprises the following steps (see Figure 5):

- preparing a flat strip 18 (whose original perimeter is shown with a dashed line);
- shaping the flat strip 18 by removing material (preferably by chemical etching or using mechanical or laser systems) to form a one-piece planar configuration of one of the joining members 4 or 5;
- deforming the shaped flat strip 18 (by bending) to obtain an annular configuration comprising the first ring and the second ring 9 and the connecting arms 10, as shown in Figure 2.

Similarly, see Figure 6, the connector 6 can also be made using the same process, that is, shaping a flat strip 20 by removing material (preferably by chemical etching) and then bending it to give the connector 6 a ring- shaped configuration.

This method has the advantage of minimizing waste, thereby reducing production costs, while at the same time allowing joints to be made very quickly.

The joint according to the invention therefore offers several advantages.

The architecture of the joining members allows the overall dimensions (especially radial dimensions) to be minimized and avoids having to evert the end portions of the duct and making the rods penetrate into the interior of the duct in contact with the fluid circulating in the duct. This is achieved by the special configuration of the joining members which allows application on the duct (with a close-packed configuration of the joining element) by plastic deformation of the arms in response to the action of moving the two rings closer together.

The possibility of having a joint with an open ring structure immediately or soon after application makes it possible for the joint to follow the pulsating or peristaltic movement of the duct even in the section where the joint is applied, thus avoiding possible narrowing of the duct passage at the joint.

The built-in pressure detecting sensor in the joint (for example applied to the connector) is designed to transmit a signal indicating the pressure of the fluid in the section where the joint is applied (this signal being detected for example in response to an elastic deformation of the joint in the open-ring embodiment of it) and allows monitoring of the state of health of the person the joint is implanted in and/or monitoring of the critical zones of the body ducts of the person (with implanted joints).

The above mentioned method of making the joint, based on planar technology to obtain the parts of the joint (for example by chemical etching) and then bending them to form the annular configuration of the joint, allows considerable savings in production costs compared to traditional technologies.

It will be understood that the invention described is susceptible of industrial application and may be modified and adapted in several ways without thereby departing from the scope of the inventive concept. Moreover, all the details of the invention may be substituted by technically equivalent elements.

## Claims

1. An end-to-end joint for joining end zones (2, 3) of a body duct, comprising:

  - a first joining member (4) and a second joining member (5) that can be associated with corresponding end zones (2, 3) of the duct to be joined;
  - a connector (6) that can be associated with the first joining member (4) and the second joining member (5) in order to connect them to each other;

  wherein the first joining member (4) and the second joining member (5) each comprise a first ring (7) equipped with a plurality of rods (8) positioned along the ring's directrices;
  **characterized in that** the first joining member (4) and the second joining member (5) each comprise:

  - a second ring (9) coaxial with the first ring (7) and having a diameter (D9) not greater than the diameter (D7) of the first ring (7), said rings (7, 9) being adapted to encompass externally one of the end zones (2, 3) of the duct;
  - at least two arms (10) each having a first end (10a) connected to the first ring (7) and a second end (10b) connected to the second ring (9) to position the rings (7, 9) at a predetermined axial distance, with the rods (8) of the first ring (7) projecting towards the second ring (9); the arms (10) being plastically deformable from an open position where the rings (7, 9) are at said predetermined axial distance, to a bent position where the rings (7, 9) are close together and the rods (8) are positioned inside the second ring (9) interacting with the rods (8) to bend them in such a way that they converge towards an axis (X) of the rings (7, 9) in order to penetrate a wall of the duct (2, 3), the bent position of the arms (10) corresponding to a clamping position of the joining member (4, 5).

2. The joint according to claim 1, wherein each arm (10) comprises two portions of equal length uninterruptedly connected to each other in such a way as to make an angle ($\alpha$) whose amplitude may vary according to the axial distance between the first ring (7) and the second ring (9).

3. The joint according to claim 2, wherein the two portions of the arms (10) lie in planes tangent to the first ring (7) and the second ring (9), the arms (10) being deformable in response to a force applied in an axial direction to the rings (7, 9) to move them towards each other in a close-packed configuration.

4. The joint according to any of the foregoing claims, wherein the free ends (8a) of the rods (8) are tapered towards the second ring (9).

5. The joint according to any of the foregoing claims, wherein the length (L8) of the rods (8) is greater than the sum of the axial extension (E9) of the second ring (9) plus twice the thickness (S10) of the arms (10).

6. The joint according to any of the foregoing claims, wherein the first ring (7), the second ring (9), the rods (8) and the arms (10) are made of the same material.

7. The joint according to any of the foregoing claims, wherein the second ring (9) comprises a plurality of openings (11) located along its circumference and passing through from one side to another in order to make the outside wall of the duct (2, 3) visible when the joining member (4, 5) is applied to the respective end zone (2, 3) of the duct.

8. The joint according to claim 7, wherein the connector (6) comprises a plurality of tabs (12) adapted to interact with the openings (11) of the second ring (9) in order to form a snap fit.

9. The joint according to any of the foregoing claims, wherein the first ring (7) and the second ring (9) of each joining member (4, 5) have a break (13, 14) in their surfaces along one directrix, forming two edges movable towards and away from each other in order to follow a pulsation of the duct (2, 3) when the joining member (4, 5) is applied to it.

10. The joint according to claim 9, wherein the connector (6) has a break (15) in its surface along one directrix, forming two edges movable towards and away from each other in order to follow a pulsation of the duct (2, 3) when the connector (6) is applied to it.

11. The joint according to any of the foregoing claims, comprising a sensor (16) for measuring the pressure of the fluid inside the duct (2, 3) and associated with the vascular joint (1).

12. The joint according to claim 11, wherein the pressure measuring sensor (16) is located on a projection (17) of the connector (6).

13. A method for making a joint, **characterized in that** it comprises the following steps:

    - preparing a flat strip (18);
    - shaping the flat strip (18) by removing material to form a one-piece planar configuration of one of the joining members (4, 5);
    - bending the shaped flat strip (18) to obtain an annular configuration comprising the first ring (7), the second ring (9) and the connecting arms (10),

    wherein said joint is end-to-end joint for joining end zones (2, 3) of a body duct, comprising a first joining member (4) and a second joining member (5) that can be associated with corresponding end zones (2, 3) of the duct to be joined; and a connector (6) that can be associated with the first joining member (4) and the second joining member (5) in order to connect them to each other; wherein the first joining member (4) and the second joining member (5) each comprise a first ring (7) equipped with a plurality of rods (8) positioned along the ring's directrices; wherein the first joining member (4) and the second joining member (5) each comprise:

    - a second ring (9) coaxial with the first ring (7) and having a diameter (D9) not greater than the diameter (D7) of the first ring (7), said rings (7, 9) being adapted to encompass externally one of the end zones (2, 3) of the duct;
    - at least two arms (10) each having a first end (10a) connected to the first ring (7) and a second end (10b) connected to the second ring (9) to position the rings (7, 9) at a predetermined axial distance, with the rods (8) of the first ring (7) projecting towards the second ring (9); the arms (10) being plastically deformable from an open position where the rings (7, 9) are at said predetermined axial distance, to a bent position where the rings (7, 9) are close together and the rods (8) are positioned inside the second ring (9) interacting with the rods (8) to bend them in such a way that they converge towards an axis (X) of the rings (7, 9) in order to penetrate a wall of the duct (2, 3), the bent position of the arms (10) corresponding to a clamping position of the joining member (4, 5).

**14.** The method according to claim 13, wherein the material is removed by chemical etching.

**Patentansprüche**

**1.** End- zu- End- Kupplungsstück zum Verkoppeln von Endbereichen (2, 3) eines Körperkanals, umfassend:

     - ein erstes Kupplungsglied (4) und ein zweites Kupplungsglied (5), die an entsprechende Endbereiche (2, 3) des zu verkoppelnden Kanals anschließbar sind;
     - ein Verbindungsstück (6), das an das erste Kupplungsglied (4) und das zweite Kupplungsglied (5) anschließbar ist, um sie miteinander zu verbinden;

wobei das erste Kupplungsglied (4) und das zweite Kupplungsglied (5) jeweils einen ersten Ring (7) umfassen, der mit einer Vielzahl von Stäben (8) ausgestattet ist, die entlang der Leitlinien des Rings positioniert sind;
**dadurch gekennzeichnet, dass** das erste Kupplungsglied (4) und das zweite Kupplungsglied (5) jeweils Folgendes umfassen:

     - einen zweiten Ring (9), der koaxial zum ersten Ring (7) ist und einen Durchmesser (D9) aufweist, der nicht größer als der Durchmesser (D7) des ersten Rings (7) ist, wobei die Ringe (7, 9) dazu geeignet sind, einen der Endbereiche (2, 3) des Kanals außerhalb zu umschließen;
     - mindestens zwei Arme (10), von denen jeder ein erstes Ende (10a), das mit dem ersten Ring (7) verbunden ist, und ein zweites Ende (10b), das mit dem zweiten Ring (9) verbunden ist, aufweist, um die Ringe (7, 9) bei einem festgelegten Achsabstand zu positionieren, wobei die Stäbe (8) des ersten Rings (7) in Richtung des zweiten Rings (9) auskragen; wobei die Arme (10) von einer offenen Position, in der die Ringe (7, 9) im festgelegten Achsabstand liegen, in eine gebogene Position, in der die Ringe (7, 9) nah zueinander sind, plastisch verformbar sind und die Stäbe (8) innerhalb des zweiten Rings (9) positioniert sind, der mit den Stäben (8) interagiert, um sie so zu biegen, dass sie in Richtung einer Achse (X) der Ringe (7, 9) konvergieren, um eine Wand des Kanals (2, 3) zu durchdringen, wobei die gebogene Position der Arme (10) einer Einklemmposition des Kupplungsglieds (4, 5) entspricht.

**2.** Kupplungsstück nach Anspruch 1, wobei jeder Arm (10) zwei Abschnitte derselben Länge umfasst, die ununterbrochen so miteinander verbunden sind, dass ein Winkel ($\alpha$) gebildet wird, dessen Weite gemäß dem Achsabstand zwischen dem ersten Ring (7) und dem zweiten Ring (9) variieren kann.

**3.** Kupplungsstück nach Anspruch 2, wobei die zwei Abschnitte der Arme (10) in Ebenen liegen, die den ersten Ring (7) und den zweiten Ring (9) tangieren, wobei die Arme (10) als Reaktion auf eine auf die Ringe (7, 9) in einer Achsenrichtung einwirkenden Kraft verformbar sind, um sie in einer dichtgepackten Konfiguration aufeinander zu zu bewegen.

**4.** Kupplungsstück nach einem der vorangehenden Ansprüche, wobei die freien Enden (8a) der Stäbe (8) konisch auf den zweiten Ring (9) zulaufen.

**5.** Kupplungsstück nach einem der vorangehenden Ansprüche, wobei die Länge (L8) der Stäbe (8) größer als die Summe der axialen Ausdehnung (E9) des zweiten Rings (9) plus zweimal die Dicke (S10) der Arme (10) ist.

**6.** Kupplungsstück nach einem der vorangehenden Ansprüche, wobei der erste Ring (7), der zweite Ring (9), die Stäbe (8) und die Arme (10) aus demselben Material gefertigt sind.

**7.** Kupplungsstück nach einem der vorangehenden Ansprüche, wobei der zweite Ring (9) eine Vielzahl von Öffnungen (11) umfasst, die entlang seines Umfangs liegen und von einer Seite zur anderen durchlaufen, um die Außenwand des Kanals (2, 3) sichtbar zu machen, wenn das Kupplungsglied (4, 5) auf den entsprechenden Endbereich (2, 3) des Kanals aufgesetzt ist.

**8.** Kupplungsstück nach Anspruch 7, wobei das Verbindungsstück (6) eine Vielzahl von Zungen (12) umfasst, die dazu geeignet sind, mit den Öffnungen (11) des zweiten Rings (9) zu interagieren, um einen Schnappverschluss zu bilden.

**9.** Kupplungsstück nach einem der vorangehenden Ansprüche, wobei der erste Ring (7) und der zweite

**EP 2 493 394 B1**

Ring (9) eines jeden Kupplungsglieds (4, 5) einen Bruch (13, 14) in ihren Oberflächen entlang einer Leitlinie aufweisen, der zwei Kanten bildet, die aufeinander zu und voneinander weg beweglich sind, um einer Pulsierung des Kanals (2, 3) zu folgen, wenn das Kupplungsglied (4, 5) darauf aufgesetzt ist.

10. Kupplungsstück nach Anspruch 9, wobei das Verbindungsstück (6) einen Bruch (15) in seiner Oberfläche entlang einer Leitlinie aufweist, der zwei Kanten bildet, die aufeinander zu und voneinander weg beweglich sind, um einer Pulsierung des Kanals (2, 3) zu folgen, wenn das Verbindungsstück (6) darauf aufgesetzt ist.

11. Kupplungsstück nach einem der vorangehenden Ansprüche, umfassend einen Sensor (16) zum Messen des Drucks des Fluids innerhalb des Kanals (2, 3), der an das Gefäßkupplungsstück (1) anschließt.

12. Kupplungsstück nach Anspruch 11, wobei sich der druckmessende Sensor (16) auf einer Auskragung (17) des Verbindungsstücks (6) befindet.

13. Verfahren zur Fertigung eines Kupplungsstücks, **dadurch gekennzeichnet, dass** es die folgenden Schritte umfasst:

- Vorbereiten eines flachen Streifens (18);
- Formen des flachen Streifens (18) durch Entfernen von Material zum Bilden einer einstückigen, planaren Konfiguration eines der Kupplungsglieder (4, 5);
- Biegen des geformten, flachen Streifens (18), um eine ringförmige Konfiguration zu erhalten, umfassend den ersten Ring (7), den zweiten Ring (9) und die Verbindungsarme (10), wobei das Kupplungsstück ein End- zu- End- Kupplungsstück zum Verkoppeln von Endbereichen (2, 3) eines Körperkanals ist, umfassend ein erstes Kupplungsglied (4) und ein zweites Kupplungsglied (5), die an entsprechende Endbereiche (2, 3) des zu verkoppelnden Kanals anschließbar sind; und ein Verbindungsstück (6), das an das erste Kupplungsglied (4) und das zweite Kupplungsglied (5) anschließbar ist, um sie miteinander zu verbinden; wobei das erste Kupplungsglied (4) und das zweite Kupplungsglied (5) jeweils einen ersten Ring (7) umfassen, der mit einer Vielzahl von Stäben (8) ausgestattet ist, die entlang der Leitlinien des Rings positioniert sind; wobei das erste Kupplungsglied (4) und das zweite Kupplungsglied (5) jeweils Folgendes umfassen:
- einen zweiten Ring (9), der koaxial zum ersten Ring (7) ist und einen Durchmesser (D9) aufweist, der nicht größer als der Durchmesser (D7) des ersten Rings (7) ist, wobei die Ringe (7, 9) dazu geeignet sind, einen der Endbereiche (2, 3) des Kanals außerhalb zu umschließen;
- mindestens zwei Arme (10), von denen jeder ein erstes Ende (10a), das an den ersten Ring (7) anschließt, und ein zweites Ende (10b), das an den zweiten Ring (9) anschließt, aufweist, um die Ringe (7, 9) bei einem festgelegten Achsabstand zu positionieren, wobei die Stäbe (8) des ersten Rings (7) in Richtung des zweiten Rings (9) auskragen; wobei die Arme (10) von einer offenen Position, in der die Ringe (7, 9) im festgelegten Achsabstand liegen, in eine gebogene Position, in der die Ringe (7, 9) nah zueinander sind, plastisch verformbar sind und die Stäbe (8) innerhalb des zweien Rings (9) positioniert sind, der mit den Stäben (8) interagiert, um sie so zu biegen, dass sie in Richtung einer Achse (X) der Ringe (7, 9) konvergieren, um eine Wand des Kanals (2, 3) zu durchdringen, wobei die gebogene Position der Arme (10) einer Einklemmposition des Kupplungsglieds (4, 5) entspricht.

14. Verfahren nach Anspruch 13, wobei das Material durch chemisches Ätzen entfernt wird.

**Revendications**

1. Joint bout à bout destiné à raccorder des zones (2, 3) d'extrémité de conduits corporels, comprenant :

- un premier élément de jonction (4) et un second élément de jonction (5) pouvant être associés aux zones d'extrémité correspondantes (2, 3) du conduit à raccorder ;
- un connecteur (6) pouvant être associé au premier élément de jonction (4) et au second élément de jonction (5) afin de les relier l'un à l'autre ;

dans lequel le premier élément de jonction (4) et le second élément de jonction (5) comprennent chacun un premier anneau (7) doté d'une pluralité de tiges (8) positionnées le long des directrices de l'anneau ; **caractérisé en ce que** le premier élément de jonction (4) et le second élément de jonction (5) comprennent chacun :

- un second anneau (9) coaxial au premier anneau (7) et ayant un diamètre (D9) qui n'est pas supérieur au diamètre (D7) du premier anneau (7), lesdits anneaux (7, 9) étant configurés pour englober par l'extérieur une des zones (2, 3) d'extrémité du conduit ;
- au moins deux bras (10) ayant chacun une première extrémité (10a) reliée au premier an-

9

neau (7) et une seconde extrémité (IOb) reliée au second anneau (9) pour positionner les anneaux (7, 9) à une distance axiale prédéterminée, avec les tiges (8) du premier anneau (7) se dirigeant vers le second anneau (9) ; les bras (10) étant plastiquement déformables d'une position ouverte où les anneaux (7, 9) sont à ladite distance axiale prédéterminée, à une position pliée où les anneaux (7, 9) se rapprochent entre eux et les tiges (8) sont positionnées à l'intérieur du second anneau (9) interagissant avec les tiges (8) pour les plier de sorte à ce qu'elles convergent vers un axe (X) des anneaux (7, 9) afin de pénétrer une paroi du conduit (2, 3), la position pliée des bras (10) correspondant à une position de serrage de l'élément de jonction (4, 5).

2. Joint selon la revendication 1, dans lequel chaque bras (10) comprend deux parties de longueur égale reliées de façon ininterrompue l'une à l'autre de sorte à former un angle ($\alpha$) dont l'amplitude peut varier selon la distance axiale entre le premier anneau (7) et le second anneau (9).

3. Joint selon la revendication 2, dans lequel les deux parties des bras (10) sont disposées le long de plans tangents par rapport au premier anneau (7) et au second anneau (9), les bras (10) étant déformables en réponse à une force appliquée dans une direction axiale par rapport aux anneaux (7, 9) pour les déplacer l'un vers l'autre dans une configuration à assemblage compact.

4. Joint selon l'une quelconque des revendications précédentes, dans lequel les extrémités libres (8a) des tiges (8) rétrécissent en direction du second anneau (9).

5. Joint selon l'une quelconque des revendications précédentes, dans lequel la longueur (L8) des tiges (8) est supérieure à la somme de l'extension axiale (E9) du second anneau (9) et du double de l'épaisseur (S10) des bras (10).

6. Joint selon l'une quelconque des revendications précédentes, dans lequel le premier anneau (7), le second anneau (9), les tiges (8) et les bras (10) sont du même matériau.

7. Joint selon l'une quelconque des revendications précédentes, dans lequel le second anneau (9) comprend une pluralité d'ouvertures (11) situées le long de sa circonférence et passant d'un côté à l'autre afin de rendre visible la paroi extérieure du conduit (2, 3) lorsque l'élément de jonction (4, 5) est appliqué à la zone (2, 3) d'extrémité correspondante du conduit.

8. Joint selon la revendication 7, dans lequel le connecteur (6) comprend une pluralité de languettes (12) prévues pour interagir avec les ouvertures (11) du second anneau (9) afin de former un montage à pression.

9. Joint selon l'une quelconque des revendications précédentes, dans lequel le premier anneau (7) et le second anneau (9) de chaque élément de jonction (4, 5) possèdent une discontinuité (13, 14) à leurs surfaces le long d'une directrice formant deux bords pouvant se rapprocher ou s'éloigner l'un de l'autre afin de suivre une pulsation du conduit (2, 3) lorsque l'élément de jonction (4, 5) lui est appliqué.

10. Joint selon la revendication 9, dans lequel le connecteur (6) possède une discontinuité (15) à sa surface le long d'une directrice, formant deux bords pouvant se rapprocher ou s'éloigner l'un de l'autre afin de suivre une pulsation du conduit (2, 3) lorsque le connecteur (6) lui est appliqué.

11. Joint selon l'une quelconque des revendications précédentes, comprenant un capteur (16) pour mesurer la pression du fluide à l'intérieur du conduit (2, 3) et associé au joint vasculaire (1).

12. Joint selon la revendication 11, dans lequel le capteur de mesure de pression (16) est situé sur une saillie (17) du connecteur (6).

13. Procédé de fabrication d'un joint, **caractérisé en ce qu'**il comprend les étapes suivantes :

   - préparation d'une bande plate (18) ;
   - façonnage de la bande plate (18) en retirant de la matière pour former une configuration planaire monobloc d'un des éléments de jonction (4, 5) ;
   - pliage de la bande plate façonnée (18) pour obtenir une configuration annulaire comprenant le premier anneau (7), le second anneau (9) et les bras de raccordement (10), dans laquelle ledit joint est un joint bout à bout destiné à raccorder des zones (2, 3) d'extrémité d'un conduit corporel, comprenant un premier élément de jonction (4) et un second élément de jonction (5) pouvant être associés aux zones d'extrémité correspondantes (2, 3) du conduit à raccorder ; et un connecteur (6) pouvant être associé au premier élément de jonction (4) et au second élément de jonction (5) afin de les relier entre eux ; dans laquelle le premier élément de jonction (4) et le second élément de jonction (5) comprennent chacun un premier anneau (7) doté d'une pluralité de tiges (8) positionnées le long des directrices de l'anneau ; dans laquelle le premier élément de jonction (4) et le second élément de jonction (5) comprennent chacun :

- un second anneau (9) coaxial au premier anneau (7) et ayant un diamètre (D9) qui n'est pas supérieur au diamètre (D7) du premier anneau (7), lesdits anneaux (7, 9) étant configurés pour englober par l'extérieur une des zones (2, 3) d'extrémité du conduit ;

- au moins deux bras (10) ayant chacun une première extrémité (10a) reliée au premier anneau (7) et une seconde extrémité (IOb) reliée au second anneau (9) pour positionner les anneaux (7, 9) à une distance axiale prédéterminée, avec les tiges (8) du premier anneau (7) se dirigeant vers le second anneau (9) ; les bras (10) étant plastiquement déformables d'une position ouverte où les anneaux (7, 9) sont à ladite distance axiale prédéterminée, à une position pliée où les anneaux (7, 9) se rapprochent entre eux et les tiges (8) sont positionnées à l'intérieur du second anneau (9) interagissant avec les tiges (8) pour les plier de sorte à ce qu'elles convergent vers un axe (X) des anneaux (7, 9) afin de pénétrer une paroi du conduit (2, 3), la position pliée des bras (10) correspondant à une position de serrage de l'élément de jonction (4, 5).

14. Procédé selon la revendication 13, dans lequel le matériau est retiré par décapage chimique.

FIG.1

FIG.2

FIG.3

FIG.4

FIG.5

FIG.6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200172232 A
- WO 2006043129 A
- US 20050149075 A